# EUROPÄISCHE PATENTSCHRIFT

(11) **EP 1 716 836 B1**
(45) Veröffentlichungstag und Bekanntmachung des Hinweises auf die Patenterteilung: **19.01.2011**
(21) Anmeldenummer: 06008008.2
(22) Anmeldetag: 18.04.2006
(51) Int. Cl.: A61K 6/083

(54) **Oberflächenmodifizierte Füllstoffe**
Surface-modified fillers
Charges à surface modifiée

(30) Priorität: 27.04.2005 DE 102005019600
(43) Veröffentlichungstag der Anmeldung: 02.11.2006
(60) Teilanmeldung: 10189804.7
(73) Patentinhaber: Ivoclar Vivadent AG, 9494 Schaan (LI)
(72) Erfinder: Moszner, Norbert, 9493 Mauren (LI); Klapdohr, Simone, 83022 Rosenheim (DE); Salz, Ulrich, 88131 Lindau (DE); Zimmermann, Jörg, 8046 Zürich (CH); Rheinberger, Volker M., 9490 Vaduz (LI)
(74) Vertreter: UEXKÜLL & STOLBERG

(56) Entgegenhaltungen:
- EP-A- 0 333 503
- WO-A-2004/035649
- PAPE P G ET AL: "IMPROVEMENTS IN SILANE COUPLING AGENTS FOR MORE DURABLE BONDING AT THE POLYMER-REINFORCEMENT INTERFACE" ENGINEERING PLASTICS, RAPRA TECHNOLOGY, SHAWBURY, SHREWSBURY, SHROPSHIRE, GB, Bd. 6, Nr. 3, Januar 1993 (1993-01), Seiten 196-207, XP000382226 ISSN: 0952-6900

## Beschreibung

Die vorliegende Erfindung betrifft oberflächenmodifizierte Füllstoffe, die sich besonders zur Verwendung in Dentalmaterialien, zur Herstellung von Adhäsiven, Beschichtungen und Kompositen eignen.

Gefüllte Materialien auf der Basis von (Meth)acrylatmonomeren werden in der restaurativen Zahnheilkunde als Füllungs- und Befestigungsmaterialien, Fissurenversiegler, Zemente für die Kieferorthopädie, als Beschichtungsmaterialien und als Adhäsive eingesetzt. Die hierbei verwendeten Füllstoffe lassen sich in organische und anorganische Füllstoffe einteilen, wobei meistens anorganische Füllstoffe zum Einsatz kommen. Diese können wiederum in oxidische und nicht oxidische Füllstoffe unterteilt werden. Die oxidischen Füllstoffe klassifiziert man dann nochmals in silikatische und nichtsilikatische Füllstoffe.

Zu den silikatischen Füllstoffen gehören beispielsweise gemahlene Gläser, wie z.B. Barium-Silikatgläser (US 4,220,582), Strontium-Silikatgläser (DE 43 23 143) und röntgenopaque Aluminium-Fluoro-Silikatgläser, die vor allem in (meth)acrylatverstärkten Glasionomeren Verwendung finden (US 5,367,002, US 5,871,360).

Zur Verbesserung der mechanischen Eigenschaften werden die Oberflächen der Füllstoffe in der Regel so modifiziert, daß diese bei der Aushärtung des Materials durch Copolymerisation kovalent in die Polymermatrix eingebunden werden. Bei anorganischen, silikatischen Füllstoffen wird meist eine Silanisierung, d.h. eine Oberflächenmodifizierung mit vorhydrolysierten (Meth)acryloxyalkyltrialkoxysilanen, wie z.B. 3-Methacryloxypropyltrimethoxysilan, durchgeführt. Bei der Silanisierung reagiert das gebildete Silanol mit freien Si-OH-Gruppen der Füllstoffoberfläche (vgl. E. P. Plueddemann, "Silane Coupling Agents", Plenum Press, 2nd Ed., New York und London, 1991).

Die GB 1 488 403 offenbart Lithium-Aluminium-Silikat-Gläser, die mit Trimethoxy-(3-methacryloyloxypropyl)silan modifiziert werden.

Die DE 40 29 230 offenbart Füllungs- und Befestigungsmaterialien auf der Basis von Mischoxiden z.B. von Zirkonoxid und Siliciumdioxid, die mit α-Methacryloxypropyltrimethoxysilan silanisiert sein können.

Aus der US 2002/0072551 sind Fissurenversiegler bekannt, die unterschiedliche Füllstoffe; wie z.B. gemahlenes Barium- oder Lithiumaluminiumsilikatglas enthalten, das mit Silanen wie γ-Methacryloxypropyltrimethoxysilan, Dimethyldichlorsilan oder Hexamethylendisilazan oberflächenbehandelt ist.

Die US 2002/0065337 schlägt Silane wie 3-Methacryloxypropyltrimethoxysilan, 3-Methacryloxypropyldimethoxymonochlorsilan und 3-Methacryloxypropyldichlormonomethoxysilan etc. zur Silanisierung von Nanopartikeln auf der Basis von gemahlenem Glas, Kieselsäuren, Zeolithen usw. vor.

Aus der US 2004/0010055 sind Glasionomerfüllstoffe bekannt, die mit einem Silan modifiziert sind, das eine Polyalkoxyethylengruppe enthält.

Die DE 24 05 578 A1 offenbart Dentalmaterialien, die amorphe Kieselsäure mit einer maximalen Teilchengröße von 0,07 µm als Füllstoff enthalten. Der Füllstoff wird mit Trimethoxy-(3-methacryloyloxypropyl)silan behandelt.

Aus der DE 32 47 800 A1 sind amorphe, kugelförmige Mischoxide auf Basis von Silicium- und Zirkonoxid bekannt, die mit γ-Methacryloxypropyltrimethoxysilan behandelt werden und sich als Füllstoff für Zahnersatzmaterial eignen sollen.

Die DE 195 08 586 A1 offenbart polymerisierbare Dentalwerkstoffe, die als Füllstoffe kugelförmige Partikel auf der Basis von SiO₂ und Oxiden von Elementen der Gruppen I, II, III und IV des Periodensystems enthalten. Die Füllstoffpartikel können mit einer Schicht aus einem polymerisierbaren organischen Bindemittel überzogen sein. Zur Oberflächenmodifizierung werden die Partikel beispielsweise mit Trimethoxy- oder Triethoxyvinylsilan behandelt. Derartige Mischoxide werden neben der verstärkenden Wirkung zur Erhöhung der Röntgenopazität, zur Einstellung der Transparenz und zur Anpassung des Brechungsindex eingesetzt.

Aus der WO 00/69392 sind nichtsilikatische Füllstoffe auf der Basis von Zirkonoxid bekannt, die mit organofunktionellen Kopplungsmitteln wie hydrolysierbaren Methacryloyloxyzirkonaten oder Methacryloyloxyaluminozirkonaten oberflächenmodifiziert sind. Alternativ können die Kopplungsmittel über Phosphonatbindungen an die Partikeloberfläche gebunden sein. Außerdem ist eine weitere Modifizierung der Partikel mit Silanen wie Dimethylethoxyvinylsilan möglich.

Aus der WO 98/13008 ist oberflächenmodifiziertes Tantaloxid und aus der DE 100 18 405 Yttriumoxid bekannt. Diese Füllstoffe eignen sich besonders als Röntgenkontrastmittel. Aluminium- und Titanoxid dienen demgegenüber wegen ihres hohen Brechungsindex häufig als Trübungsmittel.

Nichtsilikatische Füllstoffe wie Zirkonoxid können gemäß US 6,387,981 auch durch methacrylatmodifizierte Polyethercarbonsäuren oberflächenkonditioniert werden.

Außerdem ist aus der US 6,417,244 die Oberflächenmodifizierung von nichtsilikatischen Füllstoffen mit Methacryloyloxyphosphaten bekannt.

Pape et al. ("Improvements in Silane Couplings Agents for more durable Bonding at the polymer-reinforcement Interface", Engineering Plastics, Rapra Technology, Bd. 6, Nr. 3) beschreiben Silan-haltige Kupplungsreagenzien für polymere Substrate, wie beispielsweise Füllstoffe. Die Silan-haltigen Reagenzien enthalten radikalisch polymerisierbare Gruppen.

Gemeinsam an den oben beschriebenen Füllstoffen ist, daß zur Oberflächenmodifizierung meist Haftvermittler verwendet werden, die unter sauren Bedingungen nicht stabil sind. In der Zahnheilkunde kommen jedoch immer häufiger selbstätzende, selbstkonditionierende Restaurationsmaterialien zur Anwendung, die sich dadurch auszeichnen, daß keine Präkonditionierung der Zahnhartsubstanz notwendig ist. Dazu zählen die selbstätzenden Dentin/Schmelz-Adhäsive, methacrylatverstärkte Glasionomere, selbsthaftende Komposite oder auch Compomere.

Selbstätzende Dentin/Schmelz-Adhäsive sind meist so aufgebaut, daß sie ein acides Haftmonomer, ein oder mehrere nicht saure Comonomere, Wasser oder wasserhaltige Lösungsmittelgemische, einen Polymerisationsinitiator und gegebenenfalls weitere Additive enthalten.

Die US 6,214,101 offenbart selbstätzende methacrylatverstärkte Glasionomere, die als Paste/Paste-System vorliegen. Eine der Pasten ist eine auf Wasser basierende Mischung, die eine Polysäure, wie z.B. Polyacryl- oder Polymaleinsäure, und silanmodifiziertes Bariumglaspulver enthält.

Werden in derartige selbstätzende Systeme oberflächenmodifizierte Füllstoffe der oben beschriebenen Art eingearbeitet, besteht das Problem, daß die polymerisierbaren Gruppen der Haftvermittler durch Hydrolyse abgespalten werden, so daß eine kovalente Einbindung des Füllstoffs in das Polymernetzwerk nicht mehr möglich ist. Dadurch kommt es zu einer Beeinträchtigung der verstärkenden Wirkung des Füllstoffs, und es tritt eine deutliche Reduktion der mechanischen Eigenschaften des ausgehärteten Dentalmaterials bei Wasserlagerung auf. Durch die Hydrolyse verliert der Dentalwerkstoff mit der Zeit seine klinische Tauglichkeit.

Der Erfindung liegt die Aufgabe zugrunde, Füllstoffe mit radikalisch polymerisierbaren Gruppen bereitzustellen, die gegenüber wäßrigen Säuren hydrolysestabil sind.

Die Aufgabe wird erfindungsgemäß durch Füllstoff gelöst, der oberflächlich mit einer Verbindung der Formel (I) modifiziert ist,

[(PG)-R¹-Z]ₙ-SP-[Y-R²-(AG)]ₘ (I)

in der
- AG: -SiR³R⁴X ist,
- R¹: eine C₁-C₃-Alkylengruppe oder Cyclopropylengruppe ist oder entfällt,
- R²: eine C₁-C₁₀-Alkylengruppe ist oder entfällt,
- R³: eine C₁-C₈-Alkylgruppe, Chlor oder OR⁵ ist,
- R⁴: eine C₁-C₈-Alkylgruppe, Phenyl, Chlor oder OR⁵ ist,
- R⁵: eine C₁-C₆-Alkylgruppe ist,
- X: OR⁵ oder Chlor ist,
- Y: entfällt,
- Z: CO-NR⁶, wobei R⁶ H oder C₁-C₆-Alkyl ist,
- PG: eine radikalisch polymerisierbare Gruppe der Formel 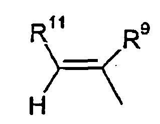 ist, in der
- R⁹: H, C₁-C₃-Alkyl, C₁-C₃-Hydroxyalkyl oder COOR¹⁰ ist,
- R¹⁰: H, C₁-C₁₀-Alkyl, 1,6-Dimethylphenyl oder Mesityl ist,
- R¹¹: H oder Phenyl ist,
- m: 1 oder 2 ist,
- n: 1, 2, 3 oder 4 ist,
- SP: entfällt oder ein (n+m)-wertiger linearer oder verzweigter aliphatischer C₁-C₃₀-Rest, bei dem die Kohlenstoffkette durch O, S, CO-NH, O-CO-NH oder NH-CO-NH unterbrochen sein kann, ein (n+m)-wertiger aromatischer C₆-C₁₈-Rest, ein (n+m)-wertiger cycloaliphatischer C₃-C₁₈-Rest oder ein (n+m)-wertiger heterocyclischer C₃-C₁₈-Rest, wobei die Reste durch C₁-C₅-Alkyl, Cl, Br und/oder OH substituiert oder unsubstituiert sein können.

Die Angabe, daß ein Rest durch Fremdatome oder Gruppen, wie Sauerstoff oder Schwefel, unterbrochen sein kann, ist so zu verstehen, daß eines oder mehrere der Fremdatome oder eine oder mehrere der Gruppen in eine Kohlenstoffkette integriert sind. Daraus folgt, daß die Fremdatome oder Gruppen nicht endständig sein können, d.h. eine Anbindung an Nachbargruppen immer über ein Kohlenstoffatom erfolgt, und daß die Zahl der Fremdatome und Gruppen zwangsläufig kleiner als die Zahl der Kohlenstoffatome sein muß.

Formel (I) erstreckt sich nur auf solche Verbindungen, die mit der chemischen Valerizlehre vereinbar sind.

PG ist vorzugsweise eine radikalisch polymerisierbare Gruppe der Formel 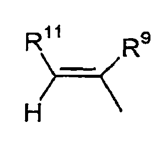 in der
- R⁹: H, CH₃, C₂H₅, Hydroxmethyl, Hydroxyethyl oder COOR¹⁰,
- R¹⁰: H, C₁-C₃-Alkyl, 1,6-Dimethylphenyl oder Mesityl,
- R¹¹: H oder Phenyl ist.

Bevorzugte polymerisationsfähige Gruppen PG sind Vinylgruppen der Formel H₂C=C(-R⁹)-, Acrylsäuregruppen der Formel H₂C=C(-COOR¹⁰)-, Allyl, Styryl und/oder Vinylcyclopropyl.

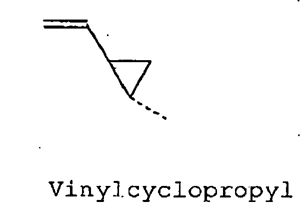

Bevorzugte Definitionen der obigen Variablen, die unabhängig voneinander gewählt werden können, sind:
- R¹ =: eine Methylengruppe, Cyclopropylengruppe oder entfällt,
- R² =: eine C₁-C₃-Alkylengruppe oder entfällt,
- R³ =: eine C₁-C₃-Alkylgruppe, Chlor, insbesondere OR⁵,
- R⁴ =: eine C₁-C₃-Alkylgruppe, Phenyl, Chlor, insbesondere OR⁵,
- R⁵ =: eine C₁-C₂-Alkylgruppe,
- R⁶ =: H, eine C₁-C₃-Alkylgruppe, insbesondere Methyl,
- X =: Chlor, insbesondere OR⁵,
- PG =: eine Vinylgruppe H₂C=C(-R⁹)-, in der R⁹ H oder CH₃ ist,
- m =: 1 oder 2,
- n =: 1 oder 2,
- SP =: entfällt oder ein (n+m)-wertiger linearer oder verzweigter aliphatischer C₁-C₆-Rest, ein (n+m)-wertiger aromatischer C₆-C₁₀-Rest, ein (n+m)-wertiger cycloaliphatischer C₃-C₁₀₋Rest oder ein (n+m)-wertiger heterocyclischer C₃-C₁₀-Rest, wobei die Reste substituiert oder unsubstituiert sein können.

Ganz besonders bevorzugt sind Verbindungen, bei denen mindestens zwei und insbesondere alle Variablen eine der bevorzugten Bedeutungen haben.

Die erfindungsgemäß als Haftvermittler eingesetzten Verbindungen der allgemeinen Formel (I) sind teilweise bekannt und zum Teil sogar kommerziell erhältlich.

Bevorzugte Verbindungen der Formel (I) (Silane) sind (Meth)acrylamidoalkyltrialkoxysilane, insbesondere 3-(N-Methacryloylamino)-propyltrimethoxysilan, 3-(N-Acryloylamino)-propyltrimethoxysilan, 3-(N-Methacryloylamino)-propyltriethoxysilan, 3-(N-Methacryl-N-ethyl-amino)-propyltrimethoxysilan, 3-(N-Methacryl-N-ethylamino)-propyltrimethoxysilan, 3-(N-Acryl-N-ethylamino)-propyltrimethoxysilan, 3-(N-Methacryl-N-methyl-amino)-propyltrimethoxysilan, 3-(N-Acryl-N-methylamino)-propyltrimethoxysilan:

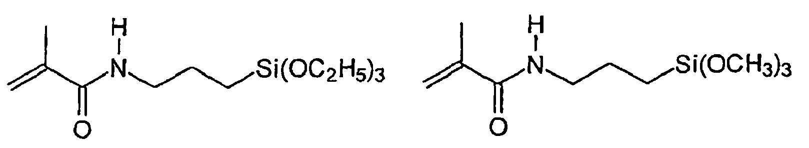

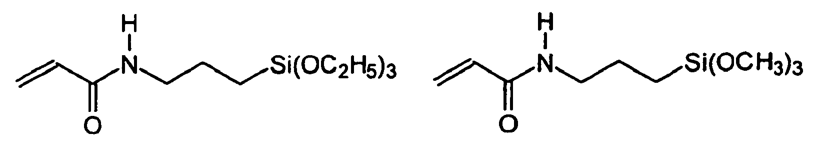

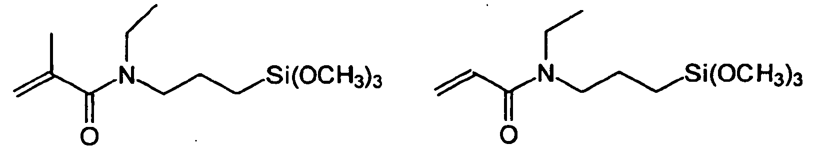

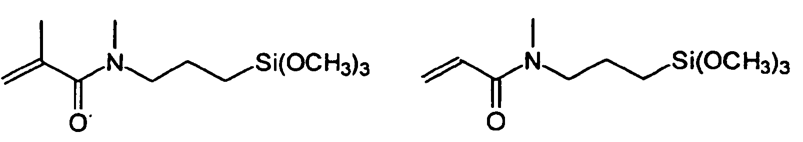

Weiter bevorzugt sind (Meth)acrylamidoalkyl-alkyl- und -aryldialkoxysilane oder (Meth)acrylamidoalkyltrichlorsilane, insbesondere 3-(N-Methacryloylamino)-propylmethyldimethoxysilan, 3-(N-Acryloylamino)-propylmethyldimethoxysilan, 3-(N-Methacryloylamino)-propylphenyldimethoxysilan, 3-(N-Acryloylamino)-propylphenyldimethoxysilan, 3-(N-Methacryl-N-ethylamino)-propyl-methyldimethoxysilan, 3-(N-Acryl-N-ethylamino)-propyl-methyldimethoxysilan, 3-(N-Methacryl-N-methyl-amino)-propyltrichlorsilan und 3-(N-Acryl-N-methylamino)-propyltrichlorsilan:

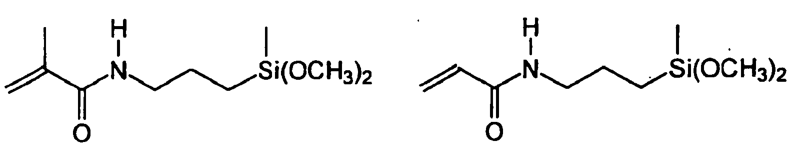

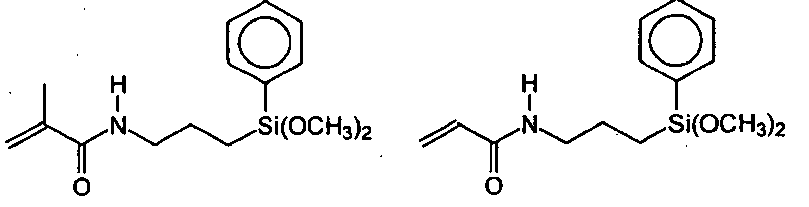

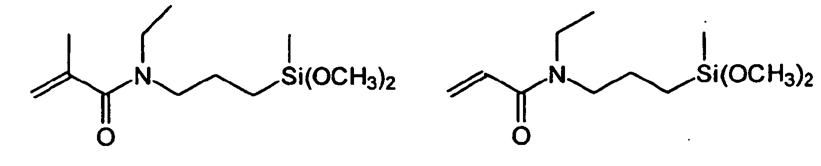

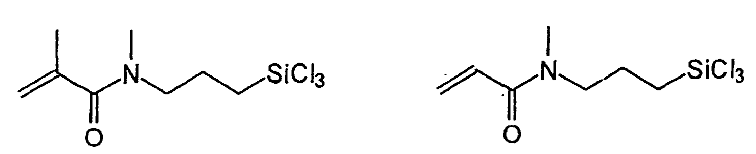

Die Oberflächenmodifizierung der Füllstoffe mit den erfindungsgemäßen Haftvermittlern der Formel (I) kann auf unterschiedlichen Wegen erfolgen.

Flüssige Haftvermittler werden vorzugsweise direkt mit den Füllstoffen vermischt, anschließend werden die Füllstoffe zur Abtrennung von Kondensationsprodukten getrocknet.

Um eine bessere Benetzung der Füllstoffoberfläche zu erreichen, ist es vor allem bei sehr feinteiligen Füllstoffen mit einer spezifischen Oberfläche von mehr als 20 oder 40 m²/g vorteilhaft, den Füllstoff in einer Lösung des Haftvermittlers in einem geeigneten Lösungsmittel zu dispergieren. Dabei läßt sich die Adsorption des Haftvermittlers durch die Füllstoffoberfläche durch die Polarität des Lösungsmittels beeinflussen. Bevorzugte Lösungsmittel sind Cyclohexan, THF, Dioxan, Ethanol und Wasser. Der Grad der Oberflächenmodifizierung hängt dabei unter anderem ab von der Füllstoffmenge und der spezifischen Oberfläche des Füllstoffs, von der Menge des Haftvermittlers, der Reaktionszeit, der Temperatur und von der Füllstoffvorbehandlung, wie z.B. von einer Vortrocknung.

Die verschiedenen Einflußfaktoren sind vor allem bei der Silanisierung von Füllstoffen sehr gut untersucht (vgl. E. P. Plueddemann, "Silane Coupling Agents", Plenum Press, 2nd Ed., New York und London, 1991; A. Guillet, Macromol. Symp. 194 (2003) 63). Bei Silanen erfolgt die Oberflächenmodifizierung durch Hydrolyse der hydrolysierbaren Gruppen am Siliciumatom durch Zugabe von Wasser und einer anschließenden Kondensation z.B. mit Hydroxylgruppen auf der Oberfläche des Füllstoffs. Im Falle von SiO₂ oder silikatischen Füllstoffen bilden sich zwischen Silanol-Gruppen der Füllstoffpartikel und Alkoxysilylgruppen der Silanhaftvermittler Siloxanbindungen aus. Die hydrolysierbaren Gruppen können auch direkt mit den Silanol-Gruppen der Partikeloberfläche unter Bildung einer Siloxanbindung reagieren. Im letzten Fall ist die Zugabe von Wasser nicht erforderlich. Oft reicht auch das Restwasser an der Füllstoffoberfläche aus. Bevorzugt wird dann die Reaktion in unpolareren Lösungsmitteln durchgeführt. Der pH-Wert beeinflußt die Hydrolyse und die Kondensation. Daher wird in der Regel ein saurer oder basischer Katalysator zugegeben. Die saure oder basische Gruppe kann aber auch schon als organischer Rest im Silan enthalten sein oder während der Hydrolyse freigesetzt werden. Um die Reaktion mit der Oberfläche zu beschleunigen kann die Suspension erwärmt werden. Durch anschließendes Verdampfen des Lösungsmittels und Trocknung des Füllstoffs kann die Kondensation weiter forciert werden. Gegebenenfalls können nicht an die Oberfläche gebundene Siloxanspezies in einem anschließenden Waschprozeß vom Füllstoff abgewaschen werden.

Nach Abschluß der Reaktion zwischen Füllstoff und Haftvermittler wird der Füllstoff abgetrennt, gegebenenfalls mit demselben oder einem anderen Lösungsmittel gewaschen, einer optionalen Wärmebehandlung unterzogen, gegebenenfalls nochmals gewaschen und dann getrocknet. Dabei kann es bei Füllstoffen, die zur Agglomeratbildung neigen, notwendig sein, diesen nach der Oberflächenmodifizierung zu mahlen oder auf andere Weise zu zerkleinern.

Bevorzugte erfindungsgemäße Füllstoffe sind solche, die auf anorganischen, partikelförmigen Füllstoffen mit einer mittleren Partikelgröße vom 0,01 bis 5 µm basieren. Diese werden erhalten, indem unbehandelte anorganische, partikelförmige Füllstoffe mit einer oder mehreren Verbindungen der Formel (I) auf die oben beschriebene Weise behandelt werden. Unter unbehandelten Füllstoffen werden Füllstoffe verstanden, die noch nicht mit Verbindungen der Formel (I) modifiziert wurden. Diese werden im folgenden auch als Ausgangsmaterialien oder Ausgangsfüllstoffe bezeichnet.

Bevorzugte Ausgangsmaterialien zur Herstellung der erfindungsgemäßen Füllstoffe sind amorpher Füllstoff auf der Basis von einem oder mehreren Metalloxiden und/oder Siliciumoxid, besonders bevorzugt unbehandelte Füllstoffe auf der Basis von ZrO₂, Ta₂O₅, TiO₂, Mischoxiden von SiO₂, ZrO₂ und/oder TiO₂, Yb₂O₃, Y₂O₃, YbF₃, Al₂O₃ und AlO(OH), Böhmit, pyrogener Kieselsäure oder Fällungskieselsäure.

Weiterhin sind Quarz-, Glaskeramik- oder Glaspulver, vorzugsweise mit einer mittleren Partikelgröße von 0,01 bis 5 µm, und röntgenopake Metallverbindungen wie Ytterbiumtrifluorid als Ausgangsfüllstoffe bevorzugt. Wenn nicht anders angegeben handelt es sich bei der mittleren Partikelgröße immer um das Gewichtsmittel, das vorzugsweise durch Lichtstreuung ermittelt wird.

Die Ausgangsfüllstoffe weisen vorzugsweise eine spezifische Oberfläche von mehr als 20 m²/g und insbesondere mehr als 40 m²/g auf, wobei Füllstoffe mit einer maximalen spezifischen Oberfläche von 300 m²/g besonders bevorzugt sind.

Die erfindungsgemäß mit einer Verbindung der Formel (I) modifizierten Füllstoffe eignen sich besonders zur Herstellung von Dentalwerkstoffen. Zur Herstellung von Dentalmaterialien werden die mit Verbindungen der Formel (I) modifizierten Füllstoffe mit einem radikalisch polymerisierbarem Bindemittel und ggf. einem Initiator für die radikalische Polymerisation gemischt.

Bevorzugte Bindemittel sind hydrolysestabile Verdünnermonomere, wie hydrolysestabilen Mono(meth)acrylaten, z.B. Mesitylmethacrylat, N-mono- oder N,N-disubstitiuierten Acrylamide, wie z.B. N-Ethylacrylamid, N,N-Dimethacrylamid, N-(2-Hydroxyethyl)acrylamid und N-Methyl-N-(2-hydroxyethyl)acrylamid, N-monosubstituierte Methacrylamide, wie z.B. N-Ethylmethacrylamid und N-(2-Hydroxyethyl)methacrylamid, sowie N-Vinylpyrrolidon.

Im Zusammenhang mit der vorliegenden Erfindung werden' unter Verdünnermonomeren solche flüssigen Monomeren mit einer oder mehreren polymerisationsfähigen Gruppen verstanden, die sich durch eine Viskosität η von kleiner 100 mPa·s (gemessen bei 20 °C) auszeichnen.

Als hydrolysestabil werden solche Monomere bezeichnet, die in Wasser oder in Mischungen von Wasser und wassermischbaren Lösungsmitteln bei einer Konzentration von ca. 20 Gew.-% und einem pH-Wert von ca. 2,0 bei 37 °C für mindestens 6 Wochen stabil sind, d.h. zu weniger als 5 % hydrolysieren.

Darüber hinaus sind hydrolysestabile Vernetzermonomere als Bindemittel bevorzugt. Unter Vernetzermonomeren werden Monomere mit zwei oder mehr, vorzugsweise 2 bis 4 radikalisch polymerisierbaren Gruppen verstanden. Bevorzugte Vernetzermonomere sind vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, Bis(meth)-acrylamide, wie z.B. N,N'-Diethyl-1,3-bis(acrylamido)-propan, 1,3-Bis(methacrylamido)-propan, 1,4-Bis(methacrylamido)-butan, 1,4-Bis(acrylamido)-butan und 1,4-Bis(acrylamido)-piperazin, die durch Umsetzung aus den entsprechenden Diaminen mit (Meth)acrylsäurechorid synthetisiert werden können. Die Vernetzermonomere können allein oder zusammen mit einem oder mehreren Verdünnermonomeren als Bindemittel eingesetzt werden.

Gemäß einer besonders bevorzugten Ausführungsform enthält das Bindemittel mindestens ein acides Monomer, im folgenden auch als Adhäsivmonomer bezeichnet. Hierbei handelt es sich um Monomere, die mindestens eine Säuregruppe, besonders bevorzugt 1 bis 4 Säuregruppen enthalten. Bevorzugte Säuregruppen sind Carbonsäure-, Sulfonsäure-, Phosphonsäure- und/oder Phosphorsäuregruppen. Verbindungen, die als acide Gruppe Carbonsäure-, Phosphonsäure- und/oder Phosphorsäuregruppen enthalten, sind besonders bevorzugt. Verbindungen mit mehr als einer Säuregruppen können unterschiedliche Säuregruppen oder vorzugsweise identischen Säuregruppen enthalten.

Für Schmelz/Dentin-Adhäsive bzw. selbsthaftende Komposite eignen sich als Adhäsivmonomere, besonders die oben beschriebenen, hydrolysestabilen, polymerisationsfähigen Acrylatetherphosphonsäuren, wie z.B. 2-[4-(Dihydroxyphosphoryl)-2-oxa-butyl]-acrylsäureethylester, 2-[4-(Dihydroxyphosphoryl)-2-oxa-butyl]-acrylsäure und 2-[4-(Dihydroxyphosphoryl)-2-oxa-butyl]-acrylsäure-2,4,6-trimethylphenylester, (Meth)acrylamidoalkylenphosphonsäuren oder -bisphosphonsäuren wie z.B. Acrylsäure-(2-phosphono-1,1-dimethylethylamin) oder Methacrylsäure-(2-phosphono-1,1-dimethylethylamin) oder N-Acrylaminomethanbisphosphonsäure.

Weiterhin sind als Adhäsivmonomere besonders auch hydrolysestabile, polymerisationsfähige Dihydrogenphosphate wie (Meth)-acrylamidoalkylen-, -cycloalkylen- oder -arylendihydrogenphosphate, z.B. 2-(N-Acryloylamino)ethyldihydrogenphosphat, 2-(N-Methacryloylamino)ethyldihydrogenphosphat, 6-(N-Acryloylamino)hexyldihydrogenphosphat, 6-(N-Methacryloylamino)hexyldihydrogenphosphat, 4-(N-Acryloylamino)phenyldihydrogenphosphat, 4-(N-Methacryloylamino)phenyldihydrogenphosphat, 1,3-Bis-(N-acryloylamino)-propan-2-yl-dihydrogenphosphat, 1,3-Bis-(N-methacryloylamino)-propan-2-yl-dihydrogenphosphat, 1,3-Bis-(N-acryloyl-N-methyl-amino)-propan-2-yl-dihydrogenphosphat oder 1,3-Bis-(N-acryloyl-N-ethyl-amino)-propan-2-yldihydrogenphosphat besonders gut geeignet.

Besonders brauchbare Adhäsivmonomere sind vor allem 2-[4-(Dihydroxyphosphoryl)-2-oxa-butyl]-acrylsäureethylester, 2-[4-(Dihydroxyphosphoryl)-2-oxa-butyl]-acrylsäure, 2-[4-(Dihydroxyphosphoryl)-2-oxa-butyl]-acrylsäure-2,4,6-trimethylphenylester, 6-(N-Acryloylamino)hexyldihydrogenphosphat, 6-(N-Methacryloylamino)hexyldihydrogenphosphat, 1,3-Bis-(N-acryloylamino)-propan-2-yl-dihydrogenphosphat, 1,3-Bis-(N-methacryloylamino)-propan-2-yl-dihydrogenphosphat.

Die aciden Monomere sind Bestandteil des Bindemittels und können sich darin im Gegensatz zu den zur Oberflächenmodifizierung der Füllstoffe eingesetzten Substanzen frei bewegen. Die an die Füllstoffoberfläche gebundenen Verbindungen weisen keine haftungsvermittelnden Eigenschaften mehr auf.

Als Bindemittel können einzelne Monomere oder Mischungen von zwei oder mehr Monomeren eingesetzt werden. Vorzugsweise enthält das Bindemittel mindestens ein Vernetzermonomer; Bindemittel, die ausschließlich vernetzende Monomere enthalten, sind besonders bevorzugt. Gemäß einer besonders bevorzugten Ausführungsform enthält das Bindemittel mindestens ein acides Monomer.

Füllstoff und Bindemittel werden allein oder unter Zugabe von einem oder mehreren Lösungsmitteln miteinander gemischt, wobei Aceton, Isopropanol und Ethanol als Lösungsmittel bevorzugt sind. Anschließend werden gegebenenfalls der Initiator und weitere Additive zugegeben.

Die erfindungsgemäßen Dentalmaterialien lassen sich durch radikalische Polymerisation aushärten. Je nach Wahl des Initiators kann die Härtung durch photochemische oder redoxinduzierte radikalische Polymerisation erfolgen.

Beispiele für bevorzugte Photoinitiatoren sind Benzophenon, Benzoin sowie deren Derivate oder α-Diketone öder deren Derivate wie 9,10-Phenanthrenchinon, Diacetyl oder 4,4-Dichlorbenzil. Besonders bevorzugt werden Campherchinon und 2,2-Dimethoxy-2-phenyl-acetophenon und besonders bevorzugt α-Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Als Initiatoren für eine bei Raumtemperatur durchgeführte Polymerisation werden Redox-Initiatorkombinationen, vorzugsweise Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und solchen Reduktionsmittel, wie z.B. Ascorbinsäure, Barbiturate oder Sulfinsäuren, besonders geeignet.

Besonders bevorzugt sind Dentalwerkstoffe, welche die folgenden Komponenten enthalten:
(a) 5 bis 90 Gew.-%, insbesondere 5 bis 50 Gew.-% Füllstoff, der mit Haftvermittler der Formel (I) oberflächenmodifiziert ist;
(b) 9,9 bis 90 Gew.-%, insbesondere 0 bis 40 Gew.-%, radikalisch polymerisierbares Monomer (Bindemittel);
(c) 0,1 bis 5,0 Gew.-%, insbesondere 0,2 bis 2,0 Gew.-%, Initiator für die radikalische Polymerisation, und/oder
(d) 0 bis 70 Gew.-%, insbesondere 0 bis 50 Gew.-%, Lösungsmittel.

Alle Prozentangaben beziehen sich, wenn nicht anders angegeben, auf die Gesamtmasse des Dentalwerkstoffs.

Der Dentalwerkstoff kann einen oder mehrere Füllstoffe enthalten, die mit einer Verbindung der Formel (I) modifiziert sind. Es ist jedoch bevorzugt, daß alle Füllstoffkomponenten des Dentalwerkstoffs in der angegebenen Weise modifiziert sind.

Acide Monomere werden vorzugsweise in einer Menge von 0 bis 70 Gew.-%, besonders bevorzugt 0 bis 50 Gew.-% und ganz besonders bevorzugt 3 bis 30 Gew.-% eingesetzt, bezogen auf die Gesamtmasse des Werkstoffs.

Die Dentalwerkstoffe eignen sich besonders als Adhäsive, Füllungskomposite, Befestigungszemente, Fissurenversiegler oder Beschichtungsmaterialien.

Die Erfindung wird im Folgenden anhand von Beispielen näher erläutert.

### Ausführungsbeispiele

### Beispiel 1: Synthese von 3-(Methacrylamido)propyltrimethoxysilan

Unter Argon wurden 77.4 g 3-(Amino)propyltrimethoxysilan, 43.7 g Triethylamin und 25 mg Di-*tert*.-butyl-*p*-kresol in 500 ml Dichlormethan gelöst. Bei -5 °C wurden 45.1 g Methacryloylchlorid langsam innerhalb 1 h zugetropft, anschließend wurde für 1 h bei 0 °C gerührt. Das ausgefallene Hydrochlorid wurde abfiltriert und mit Dichlormethan gewaschen. Die flüchtigen Bestandteile wurden bei 40 °C unter vermindertem Druck am Rotationsverdampfer entfernt. Zurück blieb eine gelbe Flüssigkeit mit abgeschiedenem Feststoff (Hydrochlorid). Die Fällung des Hydrochlorids wurde durch Zugabe von 150 ml Diethylether vervollständigt, der Niederschlag wurde abfiltriert und das Filtrat unter Einleitung trockener Luft bei 40 °C am Rotationsverdampfer eingeengt. Die bräunliche Flüssigkeit wurde bei 4x10⁻² mbar von restlichen flüchtigen Bestandteilen befreit, und das Rohprodukt (104.2 g) wurde bei einem Druck von 6x10⁻⁴ mbar destilliert. Das Produkt hatte dabei einen Siedepunkt von 123-125 °C. Es wurden 76.4 g Produkt als gelbliche, klare Flüssigkeit erhalten.

### Beispiel 2: Synthese von 3-((N-Methyl)methacrylamido)propyltrimethoxysilan

Die Synthese erfolge analog zu Beispiel 1. Statt 3-(Amino)propyltrimethoxysilan wurden 83.4 g 3-((N-Methyl)amino)propyltrimethoxysilan verwendet. Das Rohprodukt, 102.2 g einer gelben klaren Flüssigkeit, wurde bei einem Druck von 6x10⁻⁴ mbar mit einem Siedepunkt von 118 °C destilliert. Es wurden 87.8 g Produkt als farblose, klare Flüssigkeit erhalten.

### Beispiel 3: Silanisierung von OX50 mit 3-(Methacrylamido)propyltrimethoxy-silan

30 g pyrogene Kieselsäure mit einer Teilchengröße von 50 nm (OX 50, Fa. Degussa) wurden in 300 g Cyclohexan suspendiert. Nach Zugabe von 3.86 g 3-(Methacrylamido)propyltrimethoxysilan und 1.38 g Propylamin wurde 30 h auf 70 °C erwärmt. Die flüchtigen Bestandteile wurden am Rotationsverdampfer entfernt und das Produkt wurde für 3 d bei 50 °C getrocknet. Überschüssiges Silan/Kondensat, das nicht auf der Partikeloberfläche gebunden war, wurde durch mehrmaliges Dispergieren und anschließendes Zentrifugieren in Aceton, Ethanol und dann Cyclohexan herausgewaschen. Das silanisierte Pulver wurde am Rotationsverdampfer getrocknet. Man erhielt ein weißes Pulver.

### Beispiel 4: Silanisierung von OX50 mit 3-(Methacryloxy)propyl-trimethoxysilan (Vergleichsbeispiel)

Das Verfahren war dasselbe wie in Beispiel 3. Statt 3.86 g 3-(Methacrylamido)propyl-trimethoxysilan wurden 3.87 g 3-(Methacryloxy)propyltrimethoxysilan eingesetzt. Man erhielt ein weißes Pulver.

### Beispiel 5: Hydrolysestabilitätstest des silanisierten OX50

Es wurden jeweils 0.5 g des silaniserten OX50 (aus Beispiel 3 bzw. Vergleichsbeispiel 4) in 2 g 1 N Deuteriumchloridlösung in Deuteriumoxid suspendiert, als Referenz wurden jeweils 0.019 g Dimethylsulfoxid zugegeben. Für jedes Material wurden 5 Proben in separaten Gläschen vorbereitet. Die Suspensionen wurde ca. 1 h intensiv gerührt und anschließend gut verschlossen bis zur NMR-Analyse bei 42 °C gelagert. Die Proben wurden vor der spektroskopischen Untersuchung über Glaswolle filtriert. Die klaren Lösungen wurden jeweils mittels ¹H-NMR-Spektroskopie untersucht.

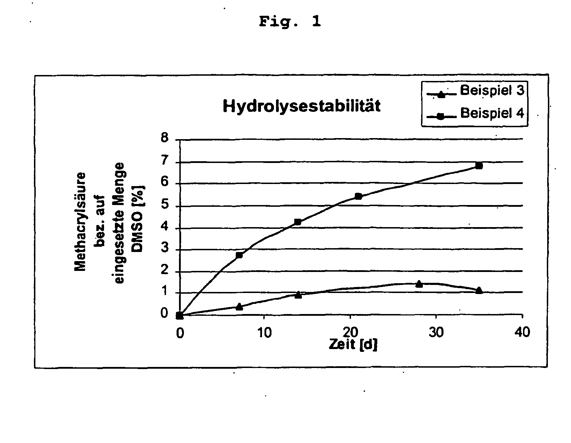

Die erste Probe wurde nach 2 h untersucht, die 2. Probe nach 1 Woche, die 3. Probe nach 3 Wochen, die 4. Probe nach 4 Wochen und die 5. Probe nach 6 Wochen. Hierzu wurde die Zunahme des Integrals des Protons der Methacrylsäuregruppe bei 5.3 ppm bezogen auf die Integral der Protonen des Phenols (MEHQ) bei 6.1-6.0 ppm beobachtet. Hieraus ließ sich die Konzentrationszunahme an Methacrylsäure, dem Hydrolyseprodukt des Silans abschätzen. Die Ergebnisse sind in Tabelle 1 zusammengefaßt und in Fig. 1 graphisch dargestellt. Der erfindungsgemäß behandelte Füllstoff weist erkennbar eine deutlich bessere Hydrolysestabilität unter sauren Bedingungen (pH < 2) auf.

**Tabelle 1: Hydroylsestabilität von silanisierten Kieselsäuren**

| **Probe** | **Zeit** | **NMR (mol% Methacrylsäure bezogen auf eingesetzte Menge DMSO)** |
|---|---|---|
| Beispiel 4 (Vergleich) | 0 d | 0 % |
| | 7 d | 2,7 % |
| | 14 d | 4,2 % |
| | 21 d | 5,4 % |
| | 35 d | 6,8 % |
| Beispiel 3 | 0 d | 0 % |
| | 7 d | 0,4 % |
| | 14 d | 0,9 % |
| | 28 d | 1,4 % |
| | 35 d | 1,1 % |

## Patentansprüche

1. Füllstoff zur Verwendung in Dentalmaterialien, **dadurch gekennzeichnet, daß** er oberflächlich mit einer Verbindung der Formel (I) modifiziert ist,
[(PG)-R¹-Z]ₙ-SP-[Y-R²-(AG)]ₘ in der (I)
AG -SiR³R⁴X ist,
R¹ eine C₁-C₃-Alkylengruppe oder Cyclopropylengruppe ist oder entfällt,
R² eine C₁-C₁₀-Alkylengruppe ist oder entfällt,
R³ eine C₁-C₈-Alkylgruppe, Chlor oder OR⁵ ist,
R⁴ eine C₁-C₈-Alkylgruppe, Phenyl, Chlor oder OR⁵ ist,
R⁵ eine C₁-C₆-Alkylgruppe ist,
X OR⁵ oder Chlor ist,
Y entfällt,
Z CO-NR⁶ ist, wobei R⁶ H oder C₁-C₆-Alkyl ist,
PG eine radikalisch polymerisierbare Gruppe der Formel 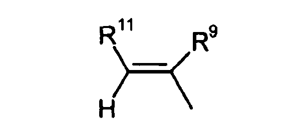 ist, in der
R⁹ H, C₁-C₃-Alkyl, C₁-C₃-Hydroxyalkyl oder COOR¹⁰ ist,
R¹⁰ H, C₁-C₁₀-Alkyl, 1,6-Dimethylphenyl oder Mesityl ist,
R¹¹ H oder Phenyl ist,
m 1 oder 2 ist,
n 1, 2, 3 oder 4 ist,
SP entfällt oder ein (n+m)-weniger linearer oder ver- zweigter aliphatischer C₁-C₃₀-Rest, bei dem die Koh- lenstoffkette durch O, S, CO-NH, O-CO-NH oder NH-CO- NH unterbrochen sein kann, ein (n+m)-wertiger aroma- tischer C₆-C₁₈-Rest, ein (n+m)-wertiger cycloaliphati- scher C₃-C₁₈-Rest oder ein (n+m)-wertiger heterocy- clischer C₃-C₁₈-Rest, wobei die Reste durch C₁-C₅- Alkyl, Cl, Br und/oder OH substituiert oder unsubsti- tuiert sein können.

2. Füllstoff nach Anspruch 1, bei dem PG eine Vinylgruppe der Formel H₂C=C(-R⁹)-, eine Acrylsäuregruppe der Formel H₂C=C(-COOR¹⁰)-, Allyl, Styryl und/oder Vinylcyclopropyl ist.

3. Füllstoff nach einem der vorhergehenden Ansprüche, bei dem die Variablen die folgenden Bedeutungen haben:
AG = -SiR³R⁴X,
R¹ = eine Methylengruppe, Cyclopropylengruppe oder entfällt,
R² = eine C₁-C₃-Alkylengruppe oder entfällt,
R³ = eine C₁-C₃-Alkylgruppe, Chlor oder OR⁵,
R⁴ = eine C₁-C₃-Alkylgruppe, Phenyl, Chlor oder OR⁵,
R⁵ = eine C₁-C₂-Alkylgruppe,
X = OR⁵ oder Chlor,
Z = CO-NR⁶,
Y = entfällt,
PG = eine Vinylgruppe H₂C=C(-R⁹)-, in der R⁹ H oder CH₃ ist,
m = 1 oder 2,
n = 1 oder 2,
SP = entfällt oder ein (n+m)-wertiger linearer oder verzweigter aliphatischer C₁-C₆-Rest, ein (n+m)-wertiger aromatischer C₆-C₁₀-Rest, ein (n+m)-wertiger cycloaliphatischer C₃-C₁₀-Rest oder ein (n+m)-wertiger heterocyclischer C₃-C₁₀-Rest, wobei die Reste durch C₁-C₅-Alkyl, Cl, Br und/oder OH substituiert oder unsubstituiert sein können.

4. Füllstoff nach einem der vorhergehenden Ansprüche, der auf anorganischem, partikelförmigem Füllstoff mit einer Partikelgröße vom 0,01 bis 5 µm basiert.

5. Füllstoff nach Anspruch 4, der ein amorpher Füllstoff auf der Basis von einem oder mehreren Metalloxiden und/oder Siliciumoxid ist.

6. Füllstoff nach Anspruch 5, der auf ZrO₂, Ta₂O₃, TiO₂, einem Mischoxid von SiO₂, ZrO₂ und/oder TiO₂, Yb₂O₃, Y₂O₃, YbF₃, Al₂O₃ und AlO(OH) Böhmit,pyrogener Kieselsäure oder Fällungskieselsäure basiert.

7. Füllstoff nach Anspruch 4, der auf Quarz-, Glaskeramik- oder Glaspulver basiert.

8. Füllstoff nach Anspruch 4, der auf einer röntgenopaken Metallverbindung wie Ytterbiumtrifluorid basiert.

9. Füllstoff nach einem der vorhergehenden Ansprüche, der auf einem anorganischem, partikelförmigen Füllstoff mit einer spezifischen Oberfläche von mehr als 20 m²/g oder mehr als 40 m²/g basiert.

10. Füllstoff nach einem der vorhergehenden Ansprüche, der dadurch erhältlich ist, daß ein anorganischer partikelförmiger Füllstoff mit einer Verbindung der Formel (I) chemisch umsetzt.

11. Dentalwerkstoff, **dadurch gekennzeichnet, daß** er einen Füllstoff gemäß einem der vorhergehenden Ansprüche enthält.

12. Dentalwerkstoff nach Anspruch 11, der
(a) 5 bis 90 Gew.-% Füllstoff nach einem der Ansprüche 1 bis 10,
(b) 9,9 bis 90 Gew.% radikalisch polymerisierbares Monomer,
(c) 0,1 bis 5,0 Gew.-% Initiator für die radikalische Polymerisation, und
(d) 0 bis 70 Gew.-% Lösungsmittel,
enthält, jeweils bezogen auf die Gesamtmasse des Dentalwerkstoffs.

13. Dentalwerkstoff nach Anspruch 12, der mindestens ein acides Monomer enthält.

14. Verwendung einer Verbindung gemäß der Formel (I) zur Oberflächenbehandlung von Füllstoffen für Dentalmaterialien.

15. Verwendung eines Füllstoffes gemäß einem der Ansprüche 1 bis 10 zur Herstellung eines Dentalwerkstoffs.

16. Verwendung nach Anspruch 15, wobei der Dentalwerkstoff ein Adhäsiv, Füllungskomposit, Befestigungszement, Fissurenversiegler oder Beschichtungsmaterial ist.

## Claims

1. A filler for use in dental materials, **characterised in that** it is surface-modified with a compound of the formula (I),
[(PG)-R¹-Z]ₙ-SP-[Y-R²-(AG)]ₘ (I)
in which
AG is -SiR³R⁴X,
R¹ is a C₁-C₃ alkylene group or cyclopropylene group or is absent,
R² is a C₁-C₁₀ alkylene group or is absent,
R³ is a C₁-C₈ alkyl group, chlorine or OR⁵,
R⁴ is a C₁-C₈ alkyl group, phenyl, chlorine or OR⁵,
R⁵ is a C₁-C₆ alkyl group,
X is OR⁵ or chlorine,
Y is absent,
Z is CO-NR⁶, R⁶ being H or C₁-C₆ alkyl,
PG is a radically polymerisable group of the formula 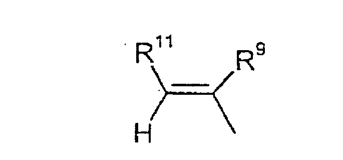 in which
R⁹ is H, C₁-C₃ alkyl, C₁-C₃ hydroxyalkyl or COOR¹⁰,
R¹⁰ is H, C₁-C₁₀ alkyl, 1,6-dimethylphenyl or mesityl,
R¹¹ is H or phenyl,
m is 1 or 2,
n is 1, 2, 3 or 4,
SP is absent or an (n+m)-valent linear or branched aliphatic C₁-C₃₀ group, in which the hydrocarbon chain can be interrupted by O, S, CO-NH, O-CO-NH or NH-CO-NH, an (n+m)-valent aromatic C₆- C₁₈ group, an (n+m)-valent cycloaliphatic C₃-C₁₈ group or an (n+m)-valent heterocyclic C₃-C₁₈ group, where the groups can be substituted by C₁-C₅ alkyl, Cl, Br and/or OH or unsubstituted.

2. The filler according to claim 1, in which PG is a vinyl group of the formula H₂C=C(-R⁹)-, an acrylic acid group of the formula H₂C=C(-COOR¹⁰)-, allyl, styryl and/or vinylcyclopropyl.

3. The filler according to one of the previous claims, in which the variables have the following meanings:
AG = -SiR³R⁴X,
R¹ = a methylene group, cyclopropylene group or is absent,
R² = a C₁-C₃ alkylene group or is absent,
R³ = a C₁-C₃ alkyl group, chlorine or OR⁵,
R⁴ = a C₁-C₃ alkyl group, phenyl, chlorine or OR⁵,
R⁵ = a C₁-C₂ alkyl group,
X = OR⁵ or chlorine,
Y = absent,
Z = CO-NR⁶,
PG = a vinyl group H₂C=C(-R⁹)-, in which R⁹ is H or CH₃,
m = 1 or 2,
n = 1 or 2,
SP = absent or an (n+m)-valent linear or branched aliphatic C₁-C₆ group, an (n+m)-valent aromatic C₆-C₁₀ group, an (n+m)-valent cycloaliphatic C₃-C₁₀ group or an (n+m)-valent heterocyclic C₃-C₁₀ group, wherein the groups can be substituted by C₁-C₅ alkyl, Cl. Br and/or OH or unsubstituted.

4. The filler according to one of the previous claims which is based on an inorganic, particulate filler with a particle size of 0.01 to 5 µm.

5. The filler according to claim 4 which is an amorphous filler based on one or more metal oxides and/or silicon oxide.

6. The filler according to claim 5 which is based on ZrO₂, Ta₂O₃, TiO₂, a mixed oxide of SiO₂, ZrO₂ and/or TiO₂, Yb₂O₃, Y₂O₃, YbF₃, Al₂O₃ and AlO(OH), bohemite, pyrogenic silica or precipitated silica.

7. The filler according to claim 4 which is based on quartz powder, glass ceramic powder or glass powder.

8. The filler according to claim 4 which is based on an X-ray opaque metal compound such as ytterbium trifluoride.

9. The filler according to one of the previous claims which is based on an inorganic, particulate filler with a specific surface area of more than 20 m²/g or more than 40 m²/g.

10. The filler according to one of the previous claims which can be obtained by chemically reacting an inorganic particulate filler with a compound of the formula (I).

11. A dental material comprising a filler according to one of the previous claims.

12. The dental material according to claim 11 comprising
(a) 5 to 90 wt.% filler according to one of claims 1 to 10,
(b) 9.9 to 90 wt.% radically polymerisable monomer,
(c) 0.1 to 5.0 wt.% initiator for the radical polymerisation, and
(d) 0 to 70 wt.% solvent,
each based on the total mass of the dental material.

13. The dental material according to claim 12 comprising at least one acidic monomer.

14. A use of a compound according to the formula (I) for the surface treatment of fillers for dental materials.

15. The use of a filler according to one of claims 1 to 10 for the preparation of a dental material.

16. The use according to claim 15, wherein the dental material is an adhesive, filling composite, fixing cement, fissure sealer or coating material.

## Revendications

1. Charge à utiliser dans des matériaux dentaires, **caractérisée en ce qu'**elle est modifiée en surface avec un composé de formule (I) :
[(PG)-R¹-Z]ₙ-SP-[Y-R²-(AG)]ₘ (I)
dans laquelle
- AG représente un groupe de formule -SiR³R⁴X,
- R¹ représente un groupe alcanediyle en C₁₋₃ ou cyclopropanediyle, ou bien ne représente rien,
- R² représente un groupe alcanediyle en C₁₋₁₀, ou bien ne représente rien,
- R³ représente un groupe alkyle en C₁₋₈, un atome de chlore ou un groupe de formule OR⁵,
- R⁴ représente un groupe alkyle en C₁₋₈ ou phényle, un atome de chlore ou un groupe de formule OR⁵,
- R⁵ représente un groupe alkyle en C₁₋₆,
- X représente un atome de chlore ou un groupe de formule OR⁵,
- Y ne représente rien,
- Z représente un raccord symbolisé par CO-NR⁶ où R⁶ représente un atome d'hydrogène ou un groupe alkyle en C₁₋₆,
- PG représente un groupe qui peut être le siège d'une polymérisation par voie radicalaire, de formule 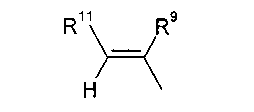 dans laquelle
- R⁹ représente un atome d'hydrogène, un groupe alkyle en C₁₋₃ ou hydroxyalkyle en C₁₋₃, ou un groupe symbolisé par COOR¹⁰,
- R¹⁰ représente un atome d'hydrogène, un groupe alkyle en C₁₋₁₀, ou un groupe 1,6-diméthyl-phényle ou mésityle,
- et R¹¹ représente un atome d'hydrogène ou un groupe phényle,
- l'indice m vaut 1 ou 2,
- l'indice n vaut 1, 2, 3 ou 4,
- et SP ne représente rien ou représente un groupe aliphatique linéaire ou ramifié en C₁₋₃₀, de valence (n + m) et dont la chaîne d'atomes de carbone peut être interrompue par un atome d'oxygène ou de soufre ou par un raccord de formule CO-NH, O-CO-NH ou NH-CO-NH, ou bien un groupe aromatique en C₆₋₁₈ de valence (n + m), un groupe cycloaliphatique en C₃₋₁₈ de valence (n + m) ou un groupe hétérocyclique en C₃₋₁₈ de valence (n + m), lesquels groupes peuvent ne porter aucun substituant ou porter un ou plusieurs substituant(s) alkyle en C₁₋₅, chloro, bromo et/ou hydroxy.

2. Charge conforme à la revendication 1, dans laquelle PG représente un groupe de type vinyle, de formule H²C=C(-R⁹)-, un groupe de type acide acrylique, de formule H²C=C(-COOR¹⁰)-, un groupe allyle, un groupe styryle et/ou un groupe vinyl-cyclopropyle.

3. Charge conforme à l'une des revendications précédentes, dans laquelle les symboles ont les significations suivantes :
- AG représente un groupe de formule -SiR³R⁴X,
- R¹ représente un groupe méthanediyle ou cyclopropanediyle, ou bien ne représente rien,
- R² représente un groupe alcanediyle en C₁₋₃, ou bien ne représente rien,
- R³ représente un groupe alkyle en C₁₋₃, un atome de chlore ou un groupe de formule OR⁵,
- R⁴ représente un groupe alkyle en C₁₋₃ ou phényle, un atome de chlore ou un groupe de formule OR⁵,
- R⁶ représente un groupe alkyle en C₁₋₂,
- X représente un atome de chlore ou un groupe de formule OR⁵,
- Z représente un raccord symbolisé par CO-NR⁶,
- Y ne représente rien,
- PG représente un groupe de type vinyle, de formule H²C=C(-R⁹)- où R⁹ représente un atome d'hydrogène ou un groupe méthyle,
- l'indice m vaut 1 ou 2,
- l'indice n vaut 1 ou 2,
- et SP ne représente rien ou représente un groupe aliphatique linéaire ou ramifié en C₁₋₆ de valence (n + m), un groupe aromatique en C₆₋₁₀ de valence (n + m), un groupe cycloaliphatique en C₃₋₁₀ de valence (n + m) ou un groupe hétérocyclique en C₃₋₁₀ de valence (n + m), lesquels groupes peuvent ne porter aucun substituant ou porter un ou plusieurs substituant(s) alkyle en C₁₋₅, chloro, bromo et/ou hydroxy.

4. Charge conforme à l'une des revendications précédentes, qui est à base d'une charge inorganique particulaire dont les particules ont une taille de 0,01 à 5 µm.

5. Charge conforme à la revendication 4, qui est une charge amorphe à base d'un ou de plusieurs oxyde(s) métallique(s) et/ou d'oxyde de silicium.

6. Charge conforme à la revendication 5, qui est une charge à base d'oxyde ZrO₂, Ta₂O₃ ou TiO₂, d'un oxyde mixte de SiO₂, ZrO₂ et/ou TiO₂, d'oxyde Yb₂O₃, Y₂O₃ ou Al₂O₃, de boehmite AlO(OH), d'acide silicique pyrogène ou d'acide silicique précipité.

7. Charge conforme à la revendication 4, qui est à base d'une poudre de quartz, de vitrocéramique ou de verre.

8. Charge conforme à la revendication 4, qui est à base d'un composé métallique opaque aux rayons X, tel le trifluorure d'ytterbium.

9. Charge conforme à l'une des revendications précédentes, qui est à base d'une charge inorganique particulaire présentant une aire spécifique de plus de 20 m²/g ou de plus de 40 m²/g.

10. Charge conforme à l'une des revendications précédentes, qu'on peut obtenir en faisant réagir chimiquement une charge inorganique particulaire avec un composé de formule (I).

11. Matériau dentaire, **caractérisé en ce qu'**il contient une charge conforme à l'une des revendications précédentes.

12. Matériau dentaire conforme à la revendication 11, qui contient, en pourcentages rapportés chacun à la masse totale du matériau dentaire :
a) 5 à 90 % en poids d'une charge conforme à l'une des revendications 1 à 10,
b) 9,9 à 90 % en poids d'un monomère polymérisable par voie radicalaire,
c) 0,1 à 5,0 % en poids d'un amorceur de polymérisation radicalaire,
d) et 0 à 70 % en poids d'un solvant.

13. Matériau dentaire conforme à la revendication 12, qui contient au moins un monomère acide.

14. Utilisation d'un composé de formule (I) pour traiter en surface des charges pour matériaux dentaires.

15. Utilisation d'une charge conforme à l'une des revendications 1 à 10 pour préparer un matériau dentaire.

16. Utilisation conforme à la revendication 15, dans laquelle le matériau dentaire est un adhésif, un composite de remplissage, un ciment de consolidation, un matériau de scellement de fissures ou un matériau de revêtement.
